# DEMANDE DE BREVET EUROPEEN

(11) **EP 1 870 160 A1**
(43) Date de publication de la demande: **26.12.2007**
(21) Numéro de dépôt: 06012481.5
(22) Date de dépôt: 19.06.2006
(51) Int. Cl.: B01L 3/00, G01N 33/53

(54) **Emballage pour outil de prélèvement de sécrétions corporelles**

(71) Demandeur: Groupe Servibio, 92190 Meudon (FR)
(72) Inventeur: Cohen, Alain, 94160 Saint Mande (FR)
(74) Mandataire: Chevalier, Renaud Philippe

(57) **Abrégé**

La présente invention se rapporte à un emballage (1) caractérisé en ce qu'il comprend au moins un compartiment de stockage apte à accueillir des moyens d'analyse (22) de sécrétions corporelles, et au moins un canal (27) le long duquel peut être glissé un outil de prélèvement (29, 30) de sécrétions corporelles en direction desdits moyens d'analyse.

## Description

La présente invention se rapporte à un emballage pour moyens d'analyse de sécrétions corporelles, ainsi qu'à un procédé d'analyse de sécrétions corporelles utilisant un tel emballage.

Il est connu depuis longtemps de procéder à des contrôles de personnes, notamment dans le cadre de compétitions sportives, en vue de déterminer si celles-ci ont préalablement absorbé ou consommé des substances illicites, comme du cannabis par exemple. Dans ce dernier cas, il est généralement prévu de mesurer la teneur de Tétra-hydro-cannabinol (THC) dans les urines contrôlées à l'aide d'un réactif approprié. Les résultats obtenus sont fiables, mais la procédure de test est relativement lente, et il est nécessaire de prévoir des locaux à proximité afin de permettre aux personnes contrôlées de s'isoler afin de produire un échantillon d'urine. La mise en oeuvre d'un tel test est donc inadaptée dans le cadre de contrôles inopinés en grand nombre pouvant être organisés par les forces de l'ordre, notamment sur le bord des routes.

Afin de pallier cet inconvénient, il a déjà été proposé de substituer des prélèvements de sécrétions corporelles, comme la salive, aux échantillons d'urine.

Une première méthode consiste à déposer une mèche équipée d'un produit réactif directement sur la langue de la personne contrôlée. Cette méthode nécessite cependant une grande quantité de salive, et demeure par conséquent peu fiable dans la mesure où la salive prélevée manque souvent de fluidité, et est chargée d'impuretés faussant d'autant plus le résultat que la quantité de salive prélevée est faible. Le problème de la quantité de salive est encore renforcé dans le cas de personnes stressées ou ayant abusé d'alcool et/ou de drogues.

Il a alors été proposé une deuxième méthode selon laquelle un tampon ouatée est introduit dans la bouche de la personne contrôlée, est frotté notamment sur les joues et la langue, puis est trempé dans un réactif durant une période de temps de l'ordre de quelques minutes. La rétention de la fumée étant plus importante au niveau des papilles gustatives que sur les joues, il est alors nécessaire de gratter la surface de la langue pour extraire un maximum de matière. Par ailleurs, outre le fait qu'une telle méthode doit également faire face aux problèmes de quantité et de mucosité de la salive prélevée, un autre inconvénient réside dans le fait que la personne qui a manipulé la tampon ouatée doit ensuite maintenir manuellement ce dernier au contact du réactif durant toute la période de temps nécessaire à la réaction. Une telle méthode est donc également inadaptée pour effectuer des contrôles fiables, rapides et en grande quantité.

Il est par ailleurs connu, notamment de US 2003/129767, de proposer un emballage comprenant au moins un compartiment de stockage apte à accueillir des moyens d'analyse de sécrétions corporelles, et au moins un canal le long duquel peut être glissé un outil de prélèvement de sécrétions corporelles en direction desdits moyens d'analyse.

La présente invention a pour but de résoudre les différents problèmes évoqués précédemment, et consiste pour cela en un emballage comprenant au moins un compartiment de stockage apte à accueillir des moyens d'analyse de sécrétions corporelles, et au moins un canal le long duquel peut être glissé un outil de prélèvement de sécrétions corporelles en direction desdits moyens d'analyse, caractérisé en ce qu'il comprend une base définissant au moins un compartiment de stockage, et au moins deux volets positionnés de part et d'autre de ladite base et pouvant chacun pivoter autour d'un axe de pliage.

Ainsi, avec un tel emballage, il est possible de prélever des sécrétions corporelles de la personne à contrôler à l'aide d'un outil de prélèvement adapté, puis de faire glisser ce dernier le long du canal jusqu'à ce qu'il entre en contact avec les moyens d'analyse desdites sécrétions corporelles. L'outil de prélèvement est alors maintenu dans cette position grâce au canal, et le manipulateur n'a par conséquent plus besoin d'assurer manuellement le maintien dudit outil durant toute la période de temps nécessaire à la réaction. Le manipulateur peut de ce fait rapidement procéder à un nouveau contrôle. De plus, après que l'outil de prélèvement a été glissé le long du canal, les problèmes liés à la quantité et à la mucosité des sécrétions corporelles prélevées peuvent être surmontés grâce au canal qui autorise l'introduction d'une ou de plusieurs gouttes de réactif d'élution permettant d'éluer le plus possible lesdites sécrétions corporelles déposées sur l'outil de prélèvement. Il en découle que le résultat obtenu lors de l'analyse est parfaitement fiable et reproductible.

L'emballage selon l'invention peut ainsi être réalisé à partir d'un corps monobloc comportant des éléments mobiles les uns par rapport aux autres. Avant utilisation, de tels corps peuvent alors être maintenus « à plat », ce qui les rend facilement stockables et transportables.

Il doit être bien compris qu'un emballage selon l'invention peut être utilisé pour l'analyse de tout type de sécrétions corporelles, notamment la salive, les larmes, la sueur, etc. Dans le cas particulier d'un test visant à détecter la consommation de cannabis, les sécrétions corporelles prélevées consisteront dans de la salive.

Avantageusement, l'emballage comprend des moyens permettant de rattacher les volets l'un à l'autre, l'un des volets étant de préférence pourvu d'au moins un ergot apte à venir se loger par complémentarité de forme dans au moins une encoche présentée par l'autre volet.

Avantageusement encore, les deux volets sont chacun équipés d'au moins un évidement principal, et ces derniers sont aptes à former un canal lorsque les deux volets sont fixés l'un dans l'autre.

Préférentiellement, en vue de faciliter l'introduction de l'outil de prélèvement dans son canal correspondant, chaque canal présente une extrémité d'introduction dont la section de passage est supérieure à la section transversale dudit outil de prélèvement.

Préférentiellement encore, chaque canal comprend au moins un tronçon intermédiaire possédant une section de passage inférieure à la section transversale de l'outil de prélèvement correspondant. En effet, étant donné que l'outil de prélèvement possède généralement une extrémité élargie élastiquement déformable sur laquelle sont déposées les sécrétions corporelles prélevées, il est alors possible de favoriser l'extraction des sécrétions corporelles par compression de ladite extrémité durant son glissement le long du tronçon intermédiaire. Les sécrétions corporelles ainsi essorées sont alors conduites grâce au canal jusqu'aux moyens d'analyse se trouvant à l'aplomb.

Avantageusement, chaque canal présente une extrémité de sortie disposée à proximité d'au moins un compartiment de stockage et possédant une section de passage supérieure à la section transversale de l'outil de prélèvement correspondant. L'extrémité élastiquement déformable de l'outil de prélèvement peut alors recouvrer sa géométrie initiale, ce qui a pour effet de caler convenablement ledit outil de prélèvement dans le canal correspondant.

Avantageusement encore, au moins deux capillaires sont ménagés de part et d'autre de chaque canal de façon diamétralement opposée. Toute fuite éventuelle de réactif d'élution est alors évitée grâce au phénomène de la capillarité, le réactif d'élution en surplus étant aspiré le long des capillaires.

Préférentiellement encore, l'emballage selon l'invention est réalisé dans un matériau transparent de façon à ce que le résultat du test puisse être vu sans nécessiter l'ouverture dudit emballage.

En outre, l'invention se rapporte également à un procédé d'analyse de sécrétions corporelles, caractérisé en ce qu'il comprend les étapes consistant à :
- prélever des sécrétions corporelles à l'aide d'au moins un outil de prélèvement,
- introduire chaque outil de prélèvement dans le canal correspondant d'un emballage selon l'invention,
- verser une ou plusieurs gouttes de réactif d'élution dans chaque canal utilisé pour éluer les sécrétions corporelles prélevées.

L'invention sera mieux comprise à l'aide de la description détaillée qui est exposée ci-dessous en regard du dessin annexé dans lequel :
La figure 1 est une vue en perspective éclatée d'un emballage selon l'invention ;
La figure 2 est une vue en perspective de l'emballage représenté à la figure 1 une fois assemblé et avant introduction d'un outil de prélèvement chargé en sécrétions corporelles ;
La figure 3 est une vue en coupe à échelle agrandie selon la ligne III-III de l'emballage représenté à la figure 2 ;
La figure 4 est une vue schématique de dessus d'un autre emballage selon l'invention en position d'ouverture ;
La figure 5 est une vue schématique de côté de l'emballage représenté à la figure 4 ;
La figure 6 est une vue schématique de côté de l'emballage représenté à la figure 1 une fois assemblé ;
La figure 7 est une vue schématique de face de l'emballage représenté à la figure 1 une fois assemblé.

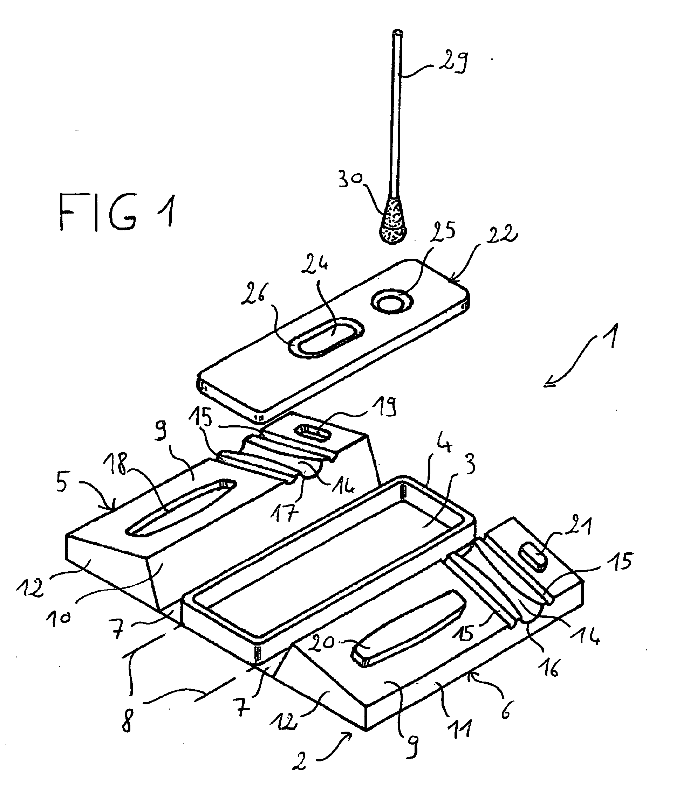

Un emballage 1 selon la présente invention, tel que représenté aux figures 1 à 3, est obtenu à partir d'un corps 2 monobloc réalisé en matière plastique.

Ce corps 2 se décompose, d'une part, en une base 3 sensiblement rectangulaire délimitée par une bordure 4, et d'autre part, en deux volets 5, 6 de mêmes dimensions disposés de part et d'autre de la base 3. Plus précisément, chaque volet 5, 6 est rattaché au grand côté correspondant de la base 3 par l'intermédiaire d'une bande 7 latérale dont la largeur est sensiblement égale à la hauteur de la bordure 4, les deux volets 5, 6 et les deux bandes 7 latérales étant conformés de façon à posséder une longueur sensiblement égale auxdits grands côtés de la base 3.

Ces deux volets 5, 6 sont chacun mobiles en rotation autour d'un axe 8 confondu avec l'arête du grand côté correspondant de la base 3. Par ailleurs, les deux volets 5, 6 se décomposent chacun en une face 9 longitudinale, un fond 10 longitudinal, un bord supérieur 11 longitudinal, et deux retours latéraux d'extrémité 12, 13.

Un évidement principal 14 sensiblement perpendiculaire au fond 10 est ménagé dans la face 9 des volets 5, 6, et est encadré par deux évidements secondaires 15 parallèles à celui-ci. L'évidement principal 14 ainsi que les deux évidements secondaires 15 associés ont une hauteur égale à celle de la face 9 dans laquelle ils sont creusés.

Chaque évidement principal 14 possède une section transversale variable, et se termine par deux extrémités 16, 17 présentant chacune une section transversale élargie. Plus précisément, la section transversale décroît régulièrement depuis chacune des deux extrémités 16, 17 en direction de l'autre extrémité 17, 16, et possède par conséquent une plus petite section transversale située à mi-distance des deux extrémités 16, 17. Par ailleurs, chacun des deux évidements secondaires 15 est semi-cylindrique et possède un diamètre de l'ordre du millimètre.

Le volet 5 possède également, d'une part, une encoche 18 principale sensiblement parallèle à l'axe 8 et située du côté du retour latéral 12, et d'autre part, une encoche 19 secondaire sensiblement perpendiculaire au fond 10 et située à proximité du retour latéral 13.

Le volet 6 est équipé d'un ergot principal 20 et d'un ergot secondaire 21 aptes respectivement à coopérer par complémentarité de formes avec les encoches 18, 19 du volet 5.

Lorsqu'un contrôle est effectué sur des personnes afin de déterminer si celles-ci ont consommées du cannabis, un membre de l'équipe procédant au test se saisit tout d'abord d'un emballage 1 préalablement stocké à plat.

Ce manipulateur se saisit ensuite de moyens d'analyse réalisés sous la forme d'une cassette 22, et place celle-ci dans le compartiment de stockage délimité par la base 3 et la bordure 4 du corps 2. Plus précisément, cette cassette 22 renferme une bande réactive 24 apte à réagir avec de la salive chargée en THC, et comporte, d'une part, un puit 25 circulaire, et d'autre part, une fenêtre de contrôle 26 bordant la zone de lecture de la bande réactive 24 en partie centrale. Dans cet exemple, la présence ou l'absence de THC se traduit respectivement au niveau de la zone de lecture par l'apparition d'une bande ou de deux bandes.

Une fois que la cassette 22 est correctement positionnée, le manipulateur fait pivoter chacun des deux volets 5, 6 autour de l'axe 8 correspondant de façon à ce que les ergots 20, 21 viennent respectivement se clipper dans les encoches 18, 19. Ce faisant, les deux évidements principaux 14 se retrouvent face à face et forment un canal 27, et de façon similaire, les évidements secondaires 15 forment deux capillaires 28 encadrant ledit canal 27.

Il doit être bien compris que l'emballage 1 et la cassette 22 sont conçus de sorte que le canal 27 soit situé à l'aplomb du puit 25 lorsque l'emballage 1 est assemblé.

Le manipulateur peut alors véritablement entamer la phase de test, et se saisit pour cela d'un écouvillon 29 possédant une extrémité ouatée 30. De manière à recueillir un maximum de salive, le manipulateur frotte avec l'extrémité ouatée 30 les parois de la cavité buccale et de la gorge, les gencives, ainsi que les papilles de la langue de la personne contrôlée.

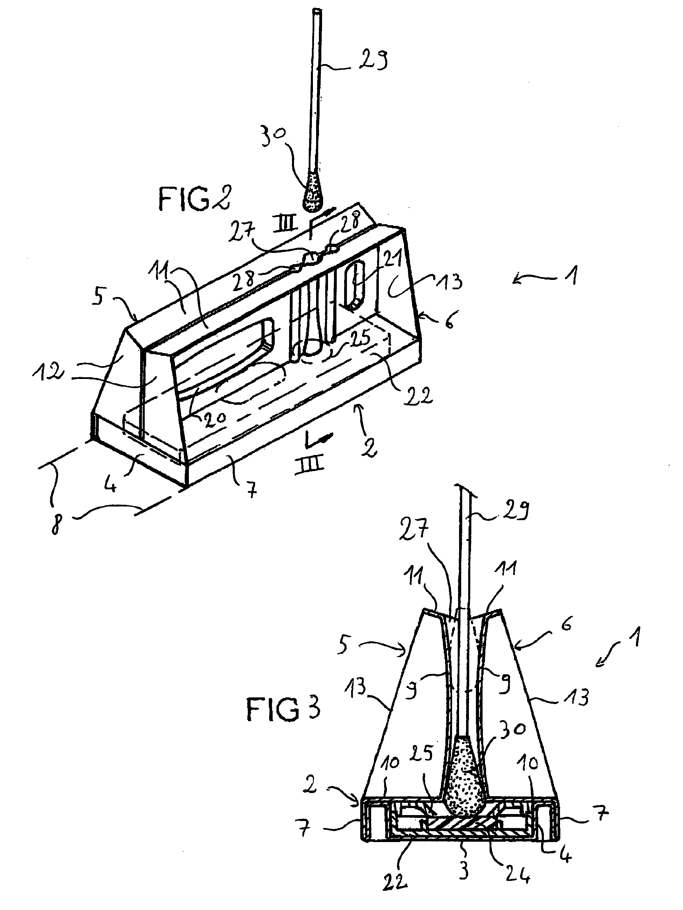

L'extrémité ouatée 30 est ensuite introduite dans l'extrémité d'introduction du canal 27, dont la section est supérieure à la section transversale de ladite extrémité ouatée 30, puis est glissée le long dudit canal 27. Comme représenté en pointillés sur la figure 3, l'extrémité ouatée 30 possédant dans cette zone une section transversale supérieure à celle du canal 27, il en découle que l'extrémité ouatée 30 est essorée dans le canal 27 au cours du glissement, et laisse de ce fait échapper une majeure partie de la salive prélevée sous la forme de gouttes (non représentée) venant s'écraser dans le puit 25 sous l'effet de la gravité.

L'extrémité ouatée 30 vient finalement au contact de la bande réactive 24 après avoir traversé l'extrémité de sortie élargie du canal 27, et recouvre de ce fait sa géométrie initiale. L'écouvillon 29 est alors efficacement maintenu en position verticale dans le canal 27. Le manipulateur peut ainsi lâcher l'écouvillon 29, puis introduire quelques gouttes de réactif d'élution (non représenté) dans le canal 27, ce qui permet en définitive d'éluer au maximum la salive prélevée par l'extrémité ouatée 30.

Les capillaires 28 permettent quant à eux d'éviter toute fuite intempestive de réactif d'élution dans la mesure où la quantité de réactif d'élution venant à déborder du puit 25 est immédiatement aspirée le long des capillaires 28 grâce au phénomène de la capillarité.

Le manipulateur peut alors déposer l'emballage 1 sur un support durant les quelques minutes nécessaires à la réaction, et procéder durant cette période de temps à un ou plusieurs autres contrôles.

Avantageusement, le matériau constitutif de l'emballage 1 est choisi transparent de façon à ce que le résultat du test puisse être vu sans nécessiter l'ouverture dudit emballage 1.

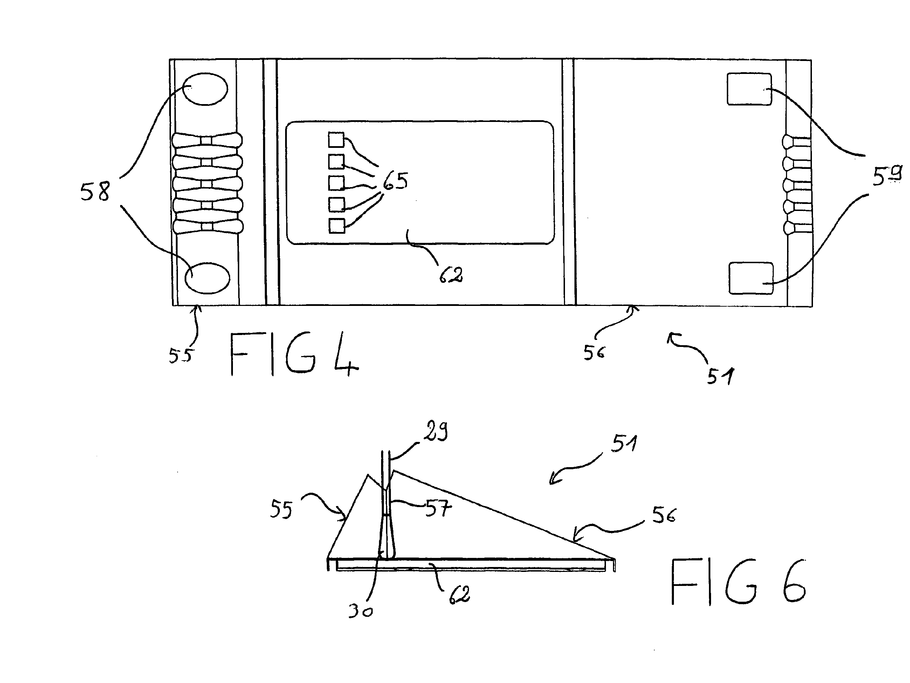

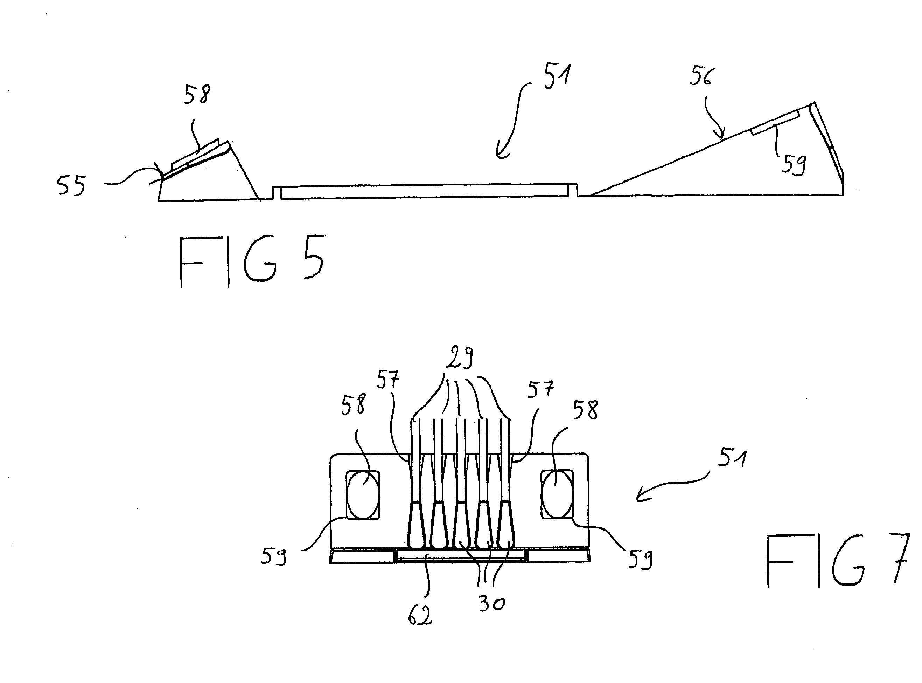

Un emballage 51 selon un autre mode de réalisation préféré de l'invention est représenté schématiquement aux figures 4 à 7, et se différencie principalement de l'emballage 1 par le fait qu'il comprend une pluralité de canaux 57. Comme précédemment, chacun de ces derniers est formé par deux évidements ménagés respectivement dans le volet 55 de petite largeur et dans le volet 56 de grande largeur, et se retrouvant face-à-face une fois que les deux ergots 58 identiques du volet 55 sont clippés dans les deux encoches 59 identiques du volet 56.

Une autre différence réside dans le fait que les moyens d'analyse sont réalisés sous la forme d'une cassette 62 possédant plusieurs puits 65, chacun de ces derniers étant conçu de façon à venir à l'aplomb du canal 57 correspondant une fois que la cassette 62 est correctement positionnée dans l'emballage 51.

L'avantage découlant d'un tel mode de réalisation consiste dans le fait qu'il est possible d'introduire plusieurs écouvillons 29 dans un seul et même emballage 51, et donc de procéder à plusieurs tests en parallèle dans le même emballage 51.

Par ailleurs, il doit être bien compris qu'un autre mode de réalisation de l'invention pourrait consister dans un emballage comprenant plusieurs compartiments de stockage permettant de loger plusieurs cassettes réactives à la même substance ou à des substances différentes (par exemple cannabis, amphétamines, ecstasy, etc.).

Bien que l'invention ait été décrite en liaison avec des exemples particuliers de réalisation, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Emballage (1, 51) comprenant au moins un compartiment de stockage apte à accueillir des moyens d'analyse (22, 62) de sécrétions corporelles, et au moins un canal (27, 57) le long duquel peut être glissé un outil de prélèvement (29, 30) de sécrétions corporelles en direction desdits moyens d'analyse, **caractérisé en ce qu'**il comprend une base (3) définissant au moins un compartiment de stockage, et au moins deux volets (5, 6 ; 55, 56) positionnés de part et d'autre de ladite basé (3) et pouvant chacun pivoter autour d'un axe de pliage (8).

2. Emballage (1) selon la revendication 1, **caractérisé en ce qu'**il comprend des moyens permettant de rattacher les volets (5, 6 ; 55, 56) l'un à l'autre.

3. Emballage (1) selon la revendication 2, **caractérisé en ce que** l'un des volets (6, 56) est pourvu d'au moins un ergot (20, 21 ; 58) apte à venir se loger par complémentarité de forme dans au moins une encoche (18, 19; 59) présentée par l'autre volet (5, 55).

4. Emballage (1) selon l'une quelconque des revendications 2 ou 3, **caractérisé en ce que** les deux volets (5, 6) sont chacun équipés d'au moins un évidement principal (14), et **en ce que** ces derniers sont aptes à former un canal (27) lorsque les deux volets sont fixés l'un dans l'autre.

5. Emballage (1, 51) selon la revendication 1, **caractérisé en ce que** chaque canal (27, 57) présente une extrémité d'introduction dont la section de passage est supérieure à la section transversale de l'outil de prélèvement (30) correspondant.

6. Emballage (1, 51) selon l'une quelconque des revendications 1 ou 5, **caractérisé en ce que** chaque canal (27, 57) comprend au moins un tronçon intermédiaire possédant une section de passage inférieure à la section transversale de l'outil de prélèvement (30) correspondant.

7. Emballage (1, 51) selon l'une quelconque des revendications 1, 5 ou 6, **caractérisé en ce que** chaque canal (27, 57) présente une extrémité de sortie disposée à proximité d'au moins un compartiment de stockage et possédant une section de passage supérieure à la section transversale de l'outil de prélèvement (30) correspondant.

8. Emballage (1) selon la revendication 1, **caractérisé en ce que** au moins deux capillaires (28) sont ménagés de part et d'autre de chaque canal (27) de façon diamétralement opposée.

9. Procédé d'analyse de sécrétions corporelles, **caractérisé en ce qu'**il consiste dans les étapes suivantes :
- prélever des sécrétions corporelles à l'aide d'au moins un outil de prélèvement (29, 30),
- introduire chaque outil de prélèvement dans le canal (27, 57) correspondant d'un emballage (1) selon l'une quelconque des revendications 1 à 8,
- verser une ou plusieurs gouttes de réactif d'élution dans le canal pour éluer les sécrétions corporelles prélevées.
